# EUROPÄISCHE PATENTANMELDUNG

(11) **EP 4 382 150 A1**
(43) Veröffentlichungstag der Anmeldung: **12.06.2024**
(21) Anmeldenummer: 22212000.8
(22) Anmeldetag: 07.12.2022
(51) Int. Cl.: A61M 5/315, A61M 5/34

(54) **KOLBEN ZUR REDUZIERUNG DES TOTVOLUMENS EINER SPRITZE**

(71) Anmelder: Transcoject GmbH, 24539 Neumünster (DE)
(72) Erfinder: Heinz, Dr. Jochen, 24220 Flintbek (DE)
(74) Vertreter: Patentanwälte Hemmer Lindfeld Frese Partnerschaft mbB

(57) **Zusammenfassung**

Die Spritze (1) weist einen Längsabschnitt (5) auf, in dem ein Kolben (10) bewegbar geführt ist, der den Spritzenzylinder (5) zu einem Ende verschließt und der mittels einer Kolbenstange (9) bewegbar ist. Distalseitig ist ein Anschluss (4) zum Aufsetzen einer Injektionsnadel (11) am anderen Ende des Spritzenzylinders (5) vorgesehen. Der Kolben (10) ist zur Reduzierung des Totvolumens über das distale Ende des Anschlusses (4) hinaus verschiebbar.

## Beschreibung

Die Erfindung betrifft ein Applikationsgefäß, insbesondere eine Spritze, mit einem Längsabschnitt, in dem ein Kolben bewegbar geführt ist, der das Applikationsgefäß zu einem Ende verschließt und der mittels einer Kolbenstange bewegbar ist. Das Applikationsgefäß weist einen Anschluss zum Aufsetzen einer Injektionsnadel am anderen Ende auf und kann ein vorgeführtes Applikationsgefäß oder ein vor der Applikation befüllbares Gefäß sein, wie dies bei Spritzen üblich ist.

Mit der zunehmenden Entwicklung empfindlicher, hochwirksamer, hochpreisiger oder auch begrenzt verfügbarer Wirkstoffe zur Injektion, die in der Regel schon bei geringer Dosierung im Bereich von Mikrolitern ihre Wirkung entfalten, besteht ein Bedarf nach Applikationsgefäßen, insbesondere Spritzen, die zum einen kleine Mengen auch bei manueller Injektion sehr genau dosieren können und bei denen zum anderen möglichst wenig Wirkstoff ungenutzt im Applikationsgefäß und der angeschlossenen Injektionsnadel zurückbleiben, die nicht weiter genutzt und zusammen mit dem Gefäß entsorgt werden müssen.

Zur Injektion kleiner Volumen ist es bekannt, Einmalspritzen zu verwenden, die einen kleinen Innendurchmesser von z.B. 4,5mm aufweisen, mit einer Feinskala versehen sind und zum Injizieren von beispielsweise 1 ml vorgesehen sind. Dabei zählt es zum Stand der Technik, zur Reduzierung des Totvolumens diese mit einem sogenannten Verdrängerstopfen auszustatten, der in den Luer-Kegel, also den Anschluss des Applikationsgefäßes, insbesondere der Spritze ragt und der die darin enthaltene Flüssigkeit verdrängt und durch die Nadel ausbringt. Zur Injektion muss jedoch eine Kanüle aufgesetzt werden, wobei das Totvolumen des Kanülensockels und der Nadel stets verbleibt. Eine darüberhinausgehende Optimierung des Totvolumens ist bei Verwendung des weltweit eingeführten Luer-Standards schwer möglich, eine Abweichung von diesem Standard würde jedoch dazu führen, dass die Spritze ihre Komptabilität zu den verfügbaren Kanülen und damit letztlich auch ihre allgemeine Gebrauchsfähigkeit verlieren würde.

Die alternativ verwendbaren Spritzen mit integrierter Nadel, bei denen die Nadel nicht aufgesetzt werden muss, sondern einstückig mit der Spritze ausgebildet ist, kann zwar mit geringerem Totvolumen konstruiert werden, allerdings ist dann die Nadel nicht austauschbar. Zudem sind die feinsten heute verfügbaren Nadeln fertigungstechnisch nur sehr schwierig mit der Spritze zu verbinden. Auch sollte die Nadel nach dem Aufziehen des Medikamentes gewechselt werden, um so eine Beschädigung der Nadelspitze, Verklebungen von Medikamenten, Unsterilitäten und dergleichen auszuschließen. Häufig sind Aufzugskanülen zur Injektion auch nicht geeignet.

Werden hingegen vorgefüllte Spritzensysteme verwendet, so kann man die vorgenannten Probleme weitgehend vermeiden. Zur präzisen Dosierung benötigt man jedoch einen möglichst geringen Durchmesser der Spritze, der über die Länge der Spritze konstant sein sollte, wobei die Spritze zur Aufnahme des nötigen Volumens eine handhabbare Länge nicht überschreiten darf. Das heute übliche minimale Füllvolumen liegt bei Glasspritzen bei 0,5ml, deren Glasrohr einen minimalen Innendurchmesser von 4,5mm aufweist und die mit einem Luer- oder Luer-Lock-Anschluss aber auch mit integrierter Nadel verfügbar sind. Dabei haben Glasspritzen bezüglich Totvolumen und Dosiergenauigkeit herstellungsbedingte Nachteile gegenüber Kunststoffspritzen, da letztere bezüglich des Innendurchmessers und der Kontur des Spritzendaches präziser gefertigt werden können. Bezüglich der Abmessungen orientieren sich jedoch auch Kunststoffspritzen wegen der Industriestandards, die sich eingebürgert haben, an den für Glasspritzen üblichen Abmessungen.

Bei vorgefüllten Spritzen mit integrierter Nadel muss die Nadelspitze dicht und steril verschlossen werden, was in der Regel durch eine Nadelkappe erfolgt. Insbesondere bei sehr dünnen Nadeln, wie sie z.B. für die Intraokularinjektion erforderlich sind, besteht dabei die Gefahr, dass die Nadel abknickt oder beschädigt wird, sodass die gesamte Spritze samt Inhalt unbrauchbar wird.

Darüber hinaus zählen Karpulen aus Glas oder aus Kunststoff zum Stand der Technik, die zur präzisen Steuerung in einen motorisch angetriebenen Vorschub integriert werden, der eine sehr genaue und reproduzierbare Dosierbarkeit erlaubt. Allerdings ist die Restentleerbarkeit bei Karpulen eher ungünstig, insbesondere wenn diese durch ein Septum verschlossen sind, welches ggf. mehrfach durch eine Nadel der zu füllenden Spritze durchstochen wird.

Vor diesem Hintergrund liegt der Erfindung die Aufgabe zugrunde, ein Applikationsgefäß, insbesondere eine Spritze der eingangs genannten Art so auszubilden, dass die vorerwähnten Probleme überwunden, insbesondere das Totvolumen beim Injizieren verringert wird.

Diese Aufgabe wird erfindungsgemäß durch ein Applikationsgefäß mit den in Anspruch 1 angegebenen Merkmalen gelöst. Vorteilhafte Ausgestaltungen der Erfindung ergeben sich aus den Unteransprüchen, der nachfolgenden Beschreibung sowie den Zeichnungen.

Das erfindungsgemäße Applikationsgefäß, insbesondere die erfindungsgemäße Spritze weist einen Längsabschnitt auf, in dem ein Kolben bewegbar geführt ist, der das Applikationsgefäß zu einem Ende verschließt und der mittels einer Kolbenstange bewegbar ist. Das Applikationsgefäß weist einen Anschluss zum Aufsetzen einer Injektionsnadel am anderen Ende des Applikationsgefäßes auf. Gemäß der Erfindung ist der Kolben zur Reduzierung des Totvolumens über das distale Ende des Anschlusses hinaus verschiebbar.

Grundgedanke der erfindungsgemäßen Lösung ist es, ein Applikationsgefäß mit Anschluss zum Aufsetzen einer Injektionsnadel und darin bewegbar geführtem Kolben so auszugestalten, dass der Kolben über das distale Ende des Anschlusses hinaus verschiebbar ist und somit das sonst im Bereich des Gegenanschlussstücks mit der Injektionsnadel vorhandene Totvolumen zumindest teilweise durch den Kolben auszufüllen, um das im Applikationsgefäß befindliche Mittel möglichst vollständig durch die Injektionsnadel hindurch auszubringen.

Applikationsgefäß im Sinne der Erfindung ist typischerweise ein Spritzenzylinder, kann aber auch ein anderes Gefäß zum Lagern und Ausbringen eines Medikaments oder eines anderen medizinischen Mittels sein. Die Querschnittsform des Längsabschnitts, in dem ein Kolben bewegbar geführt ist, ist dabei vorzugsweise aber nicht notwendigerweise kreisrund, sie kann auch oval oder drei- bzw. mehreckig und vorzugsweise mit gerundeten Ecken sein. Der Kolben, der nicht notwendigerweise eine gleiche Querschnittform, jedoch zumindest eine daran angepasste Form hat, ist dabei verschiebbar aber dichtend innerhalb des Längsabschnitts angeordnet, sodass das Gefäß, insbesondere der Spritzenzylinder an einem Ende durch den Kolben verschlossen ist und am anderen Ende den Anschluss zum Aufsetzen einer Injektionsnadel mit Gegenanschlussstück oder eines anderen Leitungsgegenanschlusses aufweist. Dabei kann der Kolben nach Überfahren des distalen Endes des gefäßseitigen Anschlusses in das Gegenanschlussstück mit der Injektionsnadel oder in den anderen Leitungsgegenanschluss zumindest eintauchen, um das dort befindliche Totvolumen zumindest teilweise zu verdrängen. Vorteilhat ist die Anordnung so, dass der Kolben das Totvolumen im Gegenanschlussstück bis zu Nadel vollständig verdrängt.

Konstruktiv erfolgt dies vorteilhaft dadurch, dass sich der Innenquerschnitt des Längsabschnitts, in dem der Kolben bewegbar geführt ist, in den Innenquerschnitt des Anschlusses fortsetzt oder kleiner als dieser ist. Bevorzugt geht die Innenwandung des Längsabschnitts bündig, vorzugsweise stufenfrei in die Innenwandung des Anschlusses über. Es versteht sich, dass bei Kunststoffgefäßen, insbesondere bei Kunststoffspritzen, welche im Spritzgussverfahren hergestellt werden, eine gewisse Konizität dieser Innenwandung stets gegeben ist, welche zum Ausformen des Werkstücks erforderlich ist. Diese Konizität ist vernachlässigbar soweit es den für die Erfindung wesentlichen vorerwähnten gleichbleibenden Querschnitt angeht.

Besonders vorteilhaft ist die Ausgestaltung des Anschlusses, insbesondere des Spritzenanschlusses als Luer- oder Luer-Lock-Anschluss, da dies wie eingangs bereits erwähnt, eine hohe Kompatibilität des erfindungsgemäßen Applikationsgefäßes mit vorhandenen Kanülen oder Leitungsanschlüssen gewährleistet. Bei Verwendung eines Luer- oder Lu-Lock-Anschlusses ist es zweckmäßig, den Kolben und den diesen umgebenden Längsabschnitt des Applikationsgefäßes im Querschnitt kreisrund auszubilden und den Innendurchmesser des Zylinders kleiner als den Außendurchmesser des (männlichen) Luer-Kegels zu gestalten, damit der Kolben das im (weiblichen) Gegenanschlussstück auf dem Luer-Kegel gebildete Totvolumen möglichst vollständig verdrängen kann.

Wenn die Innenwandung des Längsabschnittes des Applikationsgefäßes in die Innenwandung des Luer-Konus des Luer- oder Luer-Lock-Anschlusses übergeht, ergibt sich ein hohes Verdrängervolumen innerhalb des aufgesetzten Luer- oder Luer-Lock-Gegenanschlussstücks.

Dieses Verdrängervolumen kann gemäß einer vorteilhaften Weiterbildung der Erfindung noch vergrößert werden, wenn zumindest ein Abschnitt am distalen Ende des Kolbens zumindest quer zu seiner Verschieberichtung derart weichelastisch ausgebildet ist, dass er nach Verschieben des distalen Kolbenendes über das distale Ende des Anschlusses hinaus radial aufweitet. Bei einer solchen Ausbildung kann praktisch das vollständige Volumen des Gegenanschlussstücks bis hin zur Nadel bzw. der zur Nadel führenden engen Ausnehmung ausgefüllt werden. Es verbleibt dann praktisch nur das Innenvolumen der Nadel und des dahin führenden Kanals im Gegenanschlussstück als Totvolumen.

Unter weichelastisch im Sinne der Erfindung sind weiche bzw. flexible Materialien (Elastomere) zu verstehen, welche ein Elastizitätsmodul <700 MPa aufweisen. Unter hartelastisch im Sinne der vorliegenden Erfindung sind hingegen starre und halbstarre Materialien zu verstehen, wie sie in der Norm ISO 80369 definiert sind. Dabei weisen starre Materialien ein Biege- oder Zugelastizitätsmodul auf, das >3.433 MPa ist und halbstarre Materialien ein solches zwischen 700 und 3.433 MPa.

Abgesehen von der für das Entformen der Spritze ggf. erforderlichen Konizität ist es jedoch vorteilhaft, den Längsabschnitt, in welchem der Kolben bewegbar ist, mit einem gleichbleibenden Innenquerschnitt auszubilden, der sich bis in den Anschluss fortsetzt. Vorzugsweise ist dieser Längsabschnitt zylindrisch ausgebildet und weist einen kreisrunden Querschnitt auf.

Um zu verhindern, dass der Kolben in ausgefahrener Stellung eine zu große Kraft in axialer Richtung auf das Gegenanschlussstück ausübt, ist es vorteilhaft, den axialen Ausfahrweg des Kolbens in distaler Richtung zu begrenzen. Dabei erfolgt die Begrenzung zweckmäßigerweise so, dass der weichelastische Teil des Kolbens durch Einfahren in das Gegenanschlussstück das darin befindliche Totvolumen praktisch vollständig ausfüllt, ohne jedoch eine unzulässig hohe Kraft in axialer Richtung distalwärts auszuüben, welche die Verbindung zwischen Anschlussstück und Gegenanschlussstück gefährden könnte.

Vorteilhaft kann eine solche Begrenzung durch eine Rastanordnung gebildet sein, die vorzugsweise zwischen Kolben und Innenwand des Applikationsgefäßes oder zwischen Kolbenstange und dem Applikationsgefäß gebildet ist. Anstelle einer Raststellung kann auch ein Anschlag vorgesehen sein, welcher den distalen Ausfahrweg des Kolbens begrenzt.

Neben der Begrenzung des Ausfahrweges kann es vorteilhaft sein, ein oder mehrere Rastanordnungen vorzusehen, beispielsweise mit einer Raststellung, in der der Kolben das Applikationsgefäß verschließt, aber noch nicht zum dosierten Ausbringen des Gefäßinhaltes verfahren ist und/oder eine Raststellung, in welcher das distale Kolbenende das distale Ende des Luer-Anschlusses erreicht hat.

Besonders vorteilhaft ist es, wenn das Applikationsgefäß, wenn es als Spritze ausgebildet ist, am proximalen Ende eine Fingerauflage aufweist, die einen Anschlag für eine am Ende der Kolbenstange angeordnete Daumenauflage bildet. Dann ist die Handhabung zum manuellen Austragen besonders gut und es sind Fehlbedienungen praktisch ausgeschlossen.

Die erfindungsgemäße Ausgestaltung eignet sich insbesondere aber nicht nur für Spritzen mit kleinem Austragsvolumen von beispielsweise 0,3ml. Sie eignet sich dabei in hervorragender Weise als vorgefülltes Applikationsgefäß, wobei der Luer- oder Luer-Lock-Anschluss durch eine abschließende Kappe dicht verschlossen ist, die unmittelbar vor der Anwendung entfernt und durch eine mit Luer- bzw. Luer-Lock-Gegenanschluss versehene Injektionsnadel ersetzt wird.

Besonders vorteilhaft ist es, wenn die das Applikationsgefäß abschließende Kappe so ausgebildet ist, dass sie gleichzeitig die Einfahrbewegung des Kolbens blockiert, z.B. die Daumenauflage überdeckt oder aber eine gesonderte Schutzkappe vorgesehen ist, die proximalseitig des Applikationsgefäßes aufgesetzt ist und welche die Kolbenstange, insbesondere die Daumenauflage der Kolbenstange gegen eine Betätigung abschirmt. Diese Kappe ist vor Benutzung zu entfernen.

Grundsätzlich kann die erfindungsgemäße Ausgestaltung bei einer Vielzahl von hierzu geeigneten Applikationsgefäßen erfolgen, besonders vorteilhaft ist sie bei Spritzen, unabhängig davon, ob der Spritzenkörper aus Glas oder aus Kunststoff gebildet ist. Besonders vorteilhaft ist die Erfindung jedoch mit einem Spritzenkörper aus Kunststoff zu realisieren. Vorteilhaft wird die erfindungsgemäße Ausgestaltung bei einer vorgefüllten Spritze eingesetzt.

Die Erfindung ist nachfolgend anhand von in den Zeichnungen dargestellten Ausführungsbeispielen näher erläutert. Es zeigen:
- Figur 1: eine Spritze mit Luer-Lock-Anschluss im Längsschnitt,
- Figur 2: eine Spritze mit Luer-Anschluss und aufgesetztem Gegenanschlussstück mit Nadel im Längsschnitt,
- Figur 3: eine alternative Ausgestaltung in Darstellung nach Figur 2,
- Figur 4: in stark vergrößerter Längsschnittdarstellung einen gefäßseitigen Luer-Lock-Anschluss mit Innenkonus,
- Figur 5: in schematischer Darstellung eine Spritze mit Kolben in fünf unterschiedlichen Stellungen,
- Figur 6: eine Spritze mit aufgesetzter Injektionsnadel und proximaler Schutzkappe, und
- Figur 7: die Spritze gemäß Figur 6 mit aufgesetzter distaler Schutzkappe.

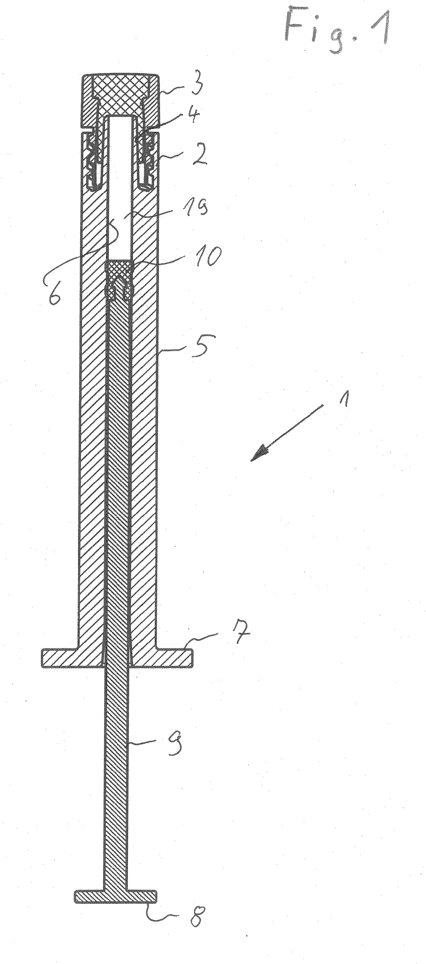

Bei der in Figur 1 dargestellten Spritze 1 handelt es sich um eine vorgefüllte Spritze mit einem distalseitigen Luer-Lock-Anschluss, der durch eine darauf sitzende Schutzkappe 3 das distale Ende der Spritze 1, insbesondere des Luer-Konus 4 dichtend abschließt. Die Spritze weist einen Spritzenzylinder 5 auf, dessen zylindrische Innenwand 6 sich bis in den Luer-Konus 4 fortsetzt, sodass eine durchgehende zylindrische Innenwand gebildet ist, welche sich durch die gesamte Spritze 1 erstreckt. Am proximalen Ende des Spritzenzylinders 5 ist eine Fingerauflage 7 angeformt, welche in Verbindung mit einer Daumenauflage 8 am proximalen Ende einer Kolbenstange 9 zum manuellen Ausbringen des im Spritzenzylinder befindlichen Mittels vorgesehen ist. Am Ende der Kolbenstange 9 sitzt der eigentliche Kolben 10, welcher axial bewegbar innerhalb des Spritzenzylinders 5 angeordnet ist und diese proximalseitig abdichtet. Spritzenzylinder 5 und Kolbenstange 9 sind auch hartelastischem Kunststoff gefertigt, wohingegen der Kolben 10 aus weichelastischem Kunststoff gefertigt ist, ebenso wie das Innere der Schutzkappe 3, welche den Luer-Konus 4 dichtend abschließt. Dabei ist die Elastizität des Materials der Gestalt, dass der Kolben 10 beim Überfahren des distalen Endes des Luer-Konus 4 radial aufweitet, um das dort bestehende Totvolumen im weiblichen Gegenanschlussstück des Luer-Konus auszufüllen.

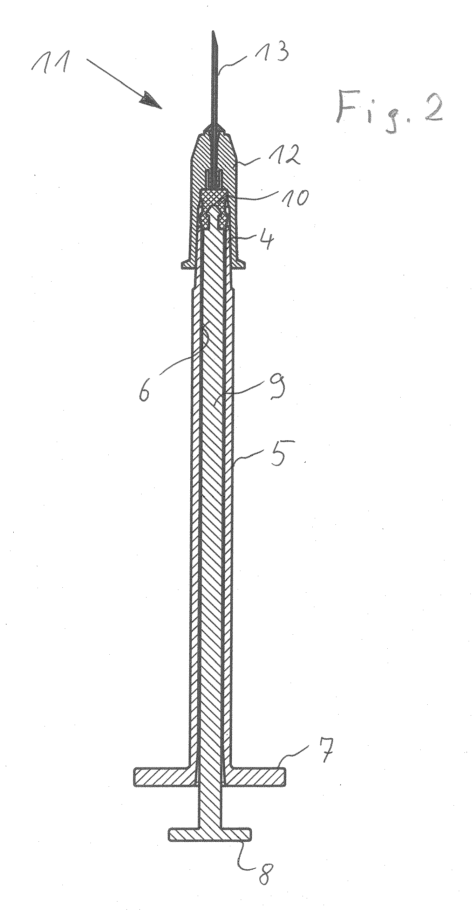

Die axiale Dimensionierung von Kolbenstange 9 mit Kolben 10 und dem Spritzenzylinder 5 mit Luer-Konus 4 ist in Figur 1 nicht maßstäblich und ist so, dass der Kolben 10 mittels der Kolbenstange 9 nach Abnehmen der Schutzkappe 3 so weit distalwärts geschoben werden kann, dass dieser distalseitig des Luer-Konus 4 vorsteht, um den in diesem Bereich bei aufgesetztem Gegenanschlussstück gebildeten Totraum auszufüllen, wie dies bei der Darstellung der Spritze gemäß Figur 2 der Fall ist. Bei der Spritze gemäß Figur 2 ist am Ende des Spritzenzylinders 5 ein Luer-Konus 4 angeformt, auf dem eine Injektionsnadel 11 aufgesetzt ist, welche mit ihrem Gegenanschlussstück 12 auf dem Luer-Konus 4 befestigt ist. Dort ist die Kolbenstange 9 mit dem distalseitig daran befestigten Kolben 10 über das distale Ende des Konus 4 hinaus bis in das Gegenanschlussstück 12 geschoben und zwar bis zu einem Absatz, in welchem sich das Gegenanschlussstück 12 zur Aufnahme der Nadel 13 verjüngt. In dieser Stellung ist der Spritzeninhalt vollständig ausgebracht und lediglich ein winzig verbleibender durch die Nadel 13 gebildeter Hohlraum noch gefüllt.

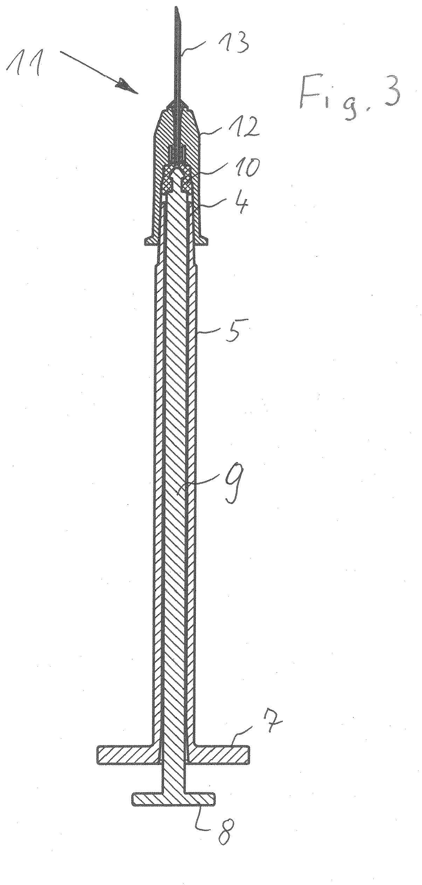

Bei der anhand von Figur 3 dargestellten Ausführungsvariante ist das Gegenanschlussstück 12 in dem den Luer-Konus 4 überragenden Bereich 14 zulaufend ausgebildet, sodass dort der weichelastische Kolben 10 das Totvolumen praktisch vollständig ausfüllen kann.

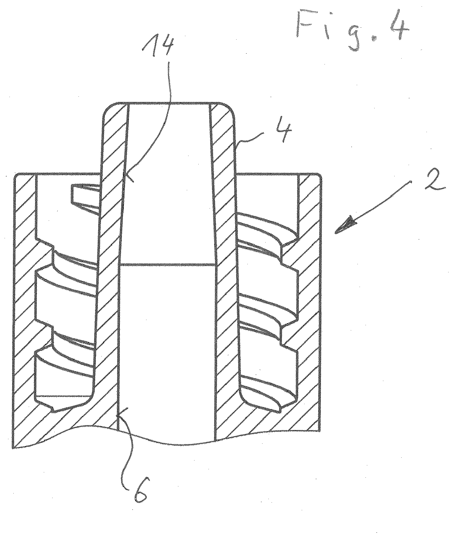

Die Schnittdarstellung gemäß Figur 4 zeigt eine spritzenseitige Ausgestaltung des Luer-Lock-Anschlusses 2, bei welcher die zylindrische Innenwand 6 im Bereich des Luer-Konus 4 in einen konisch distalwärts zulaufenden Bereich 14 übergeht. In diesem Bereich 14 wird der weichelastische Kolben 10 beim Vorschieben radial gestaucht, wodurch sich der Schiebewiderstand erhöht, sodass der Anwender spürt, dass nunmehr gleich der Kolben 10 am Ende des Luer-Konus 4 angekommen ist und dann lediglich noch das kurze Stück zum Entfernen des Totvolumens im Gegenanschlussstück 12 zu überfahren ist. Gleichzeitig führt die radiale Komprimierung dazu, dass der Kolben 10 jenseits Luer-Konus 4 radial besser aufweitet.

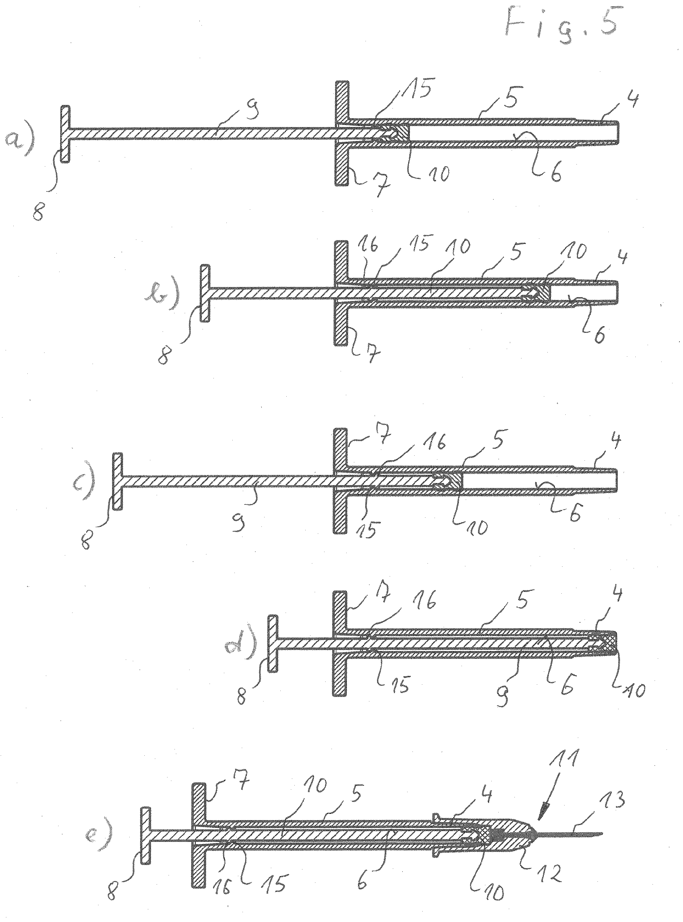

Die Handhabung einer solchen Spritze 1 kann durch geeigneter Rastanordnungen vereinfacht werden, wie dies anhand von Figur 5 dargestellt ist. Die in Figur 5a dargestellte Spritze weist eine umlaufende Rastnase 15 an der zylindrischen Innenwand 6 nahe dem proximalen Ende auf, welche so angeordnet ist, dass die Kolbenstange 9 mit dem Kolben 10 nicht vollständig aus der Spritze entfernt werden kann, sondern nur bis zum Aufziehen des maximal möglichen Volumens. Bei der Darstellung gemäß Figur 5b ist neben der umlaufenden Rastnase 15 eine damit zusammenwirkende umlaufende Rastnase 16 am Kolben 10 vorgesehen, welche eine bestimmte Stellung des Kolbens 10 markiert, beispielsweise ein vorher festgelegtes Füllvolumen.

Die Ausbildung der Rastnasen 15 und 16 ist zweckmäßigerweise derart, dass diese durch elastische Verformung nach Überwindung eines Widerstands überfahrbar sind. Bei der anhand von Figur 5c dargestellten Spritze ist eine solche Raststellung bei etwa halb eingefahrenem Kolben 10 vorgesehen. Die anhand von Figur 5d dargestellte Rastanordnung kennzeichnet die Stellung, in welcher der Kolben 10 das distale Ende des Luer-Konus 4 erreicht hat, stellt also einen Widerstand dar, der dem Behandler signalisiert, dass nunmehr nach Überwinden dieses Widerstands nur noch das Totvolumens im Gegenanschlussstück 12 auszufüllen ist.

Schließlich zeigt die Figur 5e eine Anordnung, welche als Rastanordnung aber auch als Anschlag ausgebildet sein kann, je nach radialer Erstreckung der umlaufenden Rastnasen 15 und 16. In der anhand von Figur 5e dargestellten Stellung ist es vorteilhaft statt der Rastanordnung einen Anschlag vorzusehen, da dies nämlich die Endstellung ist, in welcher der Kolben 10 über den Luer-Konus 4 distalwärts bis in das Gegenanschlussstück 12 hinaus bewegt worden ist. Er liegt dort am Absatz im Gegenanschlussstück 12 an. Durch den Anschlag kann wirksam verhindert werden, dass die axiale Kraft durch den Kolben 10 auf das Gegenanschlussstück 12 unzulässig hoch wird und den festen Sitz des Gegenanschlussstücks auf dem Luer-Konus 4 gefährden könnte.

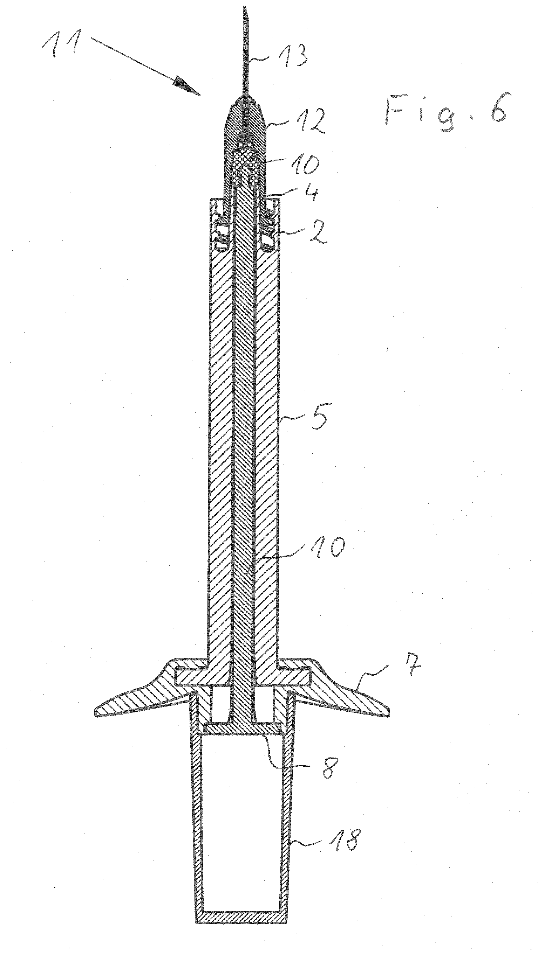

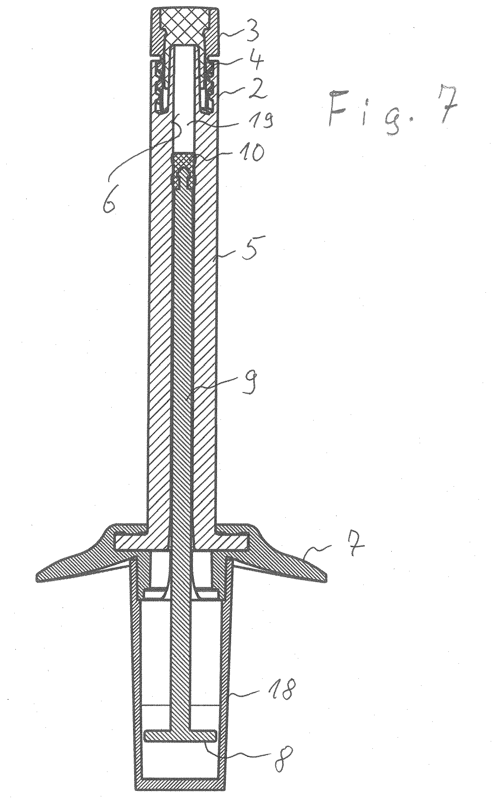

Bei der gemäß Figur 6 dargestellten Spritze, bei welcher der Kolben 10 in seiner distalen Endstellung befindlich ist, in welcher der Kolben 10 im Gegenanschlussstück 12 befindlich ist, ist der Anschlag durch die Daumenauflage 8 gebildet, welche an der dort als gesondertes Bauteil angebrachten Fingerauflage 7 anliegt. Dies verdeutlicht, dass je nach Ausgestaltung der Kolben-Zylinder-Anordnung der Anschlag nicht notwendigerweise zwischen Kolbenstange und Zylinder vorgesehen sein muss, sondern auch z.B. durch die Daumenauflage 8 am Ende der Kolbenstange 9 in Verbindung mit der Fingerauflage 7 gebildet sein kann. Die Fingerauflage 7 ist darüber hinaus proximalwärts durch einen zylindrischen Ansatz 17 verlängert, welcher zum Aufstecken einer proximalen Schutzkappe 18 vorgesehen ist, welche das proximale Ende der Kolbenstange 9 mit der Daumenauflage 8 vor einer Betätigung schützt. Diese proximale Schutzkappe 8 ist insbesondere bei einer vorgefüllten Spritze vorteilhaft, wie dies anhand von Figur 7 dargestellt ist, in welcher die Kolbenstange 9 proximalwärts ausgefahren ist und das Volumen 19 zwischen zylindrischer Innenwand 6, dem Kolben 10 und dem Ende des Luer-Konus 4, welcher durch die Schutzkappe 3 fest und dicht verschlossen ist, gebildet ist.

Wie die vorstehenden Ausführungen verdeutlichen, können einzelne Merkmale der vorliegenden Erfindung für sich, aber auch in Kombination vorteilhaft angewendet werden.

### Bezugszeichenliste

- 1: Spritze
- 2: Luer-Lock-Anschluss
- 3: Schutzkappe
- 4: Luer-Konus
- 5: Spritzenzylinder
- 6: zylindrische Innenwand
- 7: Fingerauflage
- 8: Daumenauflage
- 9: Kolbenstange
- 10: Kolben
- 11: Injektionsnadel
- 12: Gegenanschlussstück
- 13: Nadel
- 14: konisch zulaufender Bereich
- 15: Rastnase
- 16: Rastnase
- 17: zylindrischer Ansatz
- 18: proximale Schutzkappe
- 19: Volumen

## Patentansprüche

1. Applikationsgefäß, insbesondere Spritze (1), mit einem Längsabschnitt (5), in dem ein Kolben (10) bewegbar geführt ist, der das Applikationsgefäß zu einem Ende verschließt und der mittels einer Kolbenstange (9) bewegbar ist, mit einem Anschluss (4) zum Aufsetzen einer Injektionsnadel (11) am anderen Ende des Applikationsgefäßes, **dadurch gekennzeichnet, dass** der Kolben (10) zur Reduzierung des Totvolumens über das distale Ende des Anschlusses (4) hinaus verschiebbar ist.

2. Applikationsgefäß nach Anspruch 1, **dadurch gekennzeichnet, dass** der Anschluss als Luer (4)- oder Luer-Lock (2)-Anschluss ausgebildet ist.

3. Applikationsgefäß nach Anspruch 1 oder 2, **dadurch gekennzeichnet, dass** zumindest ein Abschnitt am distalen Ende des Kolbens (10) zumindest quer zu seiner Verschieberichtung derart weichelastisch ausgebildet ist, dass er nach Verschieben des distalen Kolbenendes über das distale Ende des Anschlusses (4) hinaus radial aufweitet.

4. Applikationsgefäß nach einem der vorhergehenden Ansprüche, **dadurch gekennzeichnet, dass** der Längsabschnitt (5) eine gleichbleibenden, insbesondere zylindrischen Innenquerschnitt (6) aufweist, der sich bis in den Anschluss (4) fortsetzt.

5. Applikationsgefäß nach einem der vorhergehenden Ansprüche, **dadurch gekennzeichnet, dass** der axiale Ausfahrweg des Kolbens (10) in distaler Richtung begrenzt ist.

6. Applikationsgefäß nach einem der vorhergehenden Ansprüche, **dadurch gekennzeichnet, dass** zwischen Kolben (10) und Innenwand (11) des Applikationsgefäßes oder zwischen Kolbenstange (9) und Applikationsgefäß mindestens eine Rastanordnung (15, 16) vorgesehen ist.

7. Applikationsgefäß nach Anspruch 6, **dadurch gekennzeichnet, dass** eine Rastanordnung (15, 16) mit einer Raststellung vorgesehen ist, in der der Kolben (10) das distale Ende des Luer (4)- oder Luer-Lock (2)-Anschlusses erreicht hat.

8. Applikationsgefäß nach Anspruch 6 oder 7, **dadurch gekennzeichnet, dass** eine Rastanordnung (15, 16) mit einer Raststellung vorgesehen ist, in der der Kolben (10) das Applikationsgefäß verschließt aber noch nicht zum dosierten Ausbringen des Gefäßinhaltes verfahren ist.

9. Applikationsgefäß nach einem der vorhergehenden Ansprüche, **dadurch gekennzeichnet, dass** ein Anschlag (15) für den Kolben (10) oder die Kolbenstange (9) vorgesehen ist, welcher den Verfahrweg des Kolbens (10) distalwärts begrenzt.

10. Applikationsgefäß nach Anspruch 9, **dadurch gekennzeichnet, dass** der Anschlag (15) so angeordnet ist, dass er den Verfahrweg des Kolbens (10) vor oder bis zum Erreichen eines Absatzes des Kanülensockels (12) einer auf den Luer (4)- oder Luer-Lock (2)-Anschluss aufgesetzten Kanüle (11) begrenzt.

11. Applikationsgefäß nach einem der vorhergehenden Ansprüche, **dadurch gekennzeichnet, dass** am proximalen Ende des Applikationsgefäßes eine Fingerauflage (7) angeordnet ist, die einen Anschlag für eine am Ende der Kolbenstange (9) angeordnete Daumenauflage (8) bildet.

12. Applikationsgefäß nach einem der vorhergehenden Ansprüche, **dadurch gekennzeichnet, dass** das Applikationsgefäß vorgefüllt und mit einer den Luer (4)- oder Luer-Lock (2)-Anschluss abschließenden Kappe (3) versehen ist.

13. Applikationsgefäß nach einem der vorhergehenden Ansprüche, **dadurch gekennzeichnet, dass** eine Schutzkappe (18) vorgesehen ist, die proximalseitig des Applikationsgefäßes aufgesetzt ist und welche die Daumenauflage (8) der Kolbenstange (9) gegen eine Betätigung abschirmt.

14. Applikationsgefäß nach einem der vorhergehenden Ansprüche, **dadurch gekennzeichnet, dass** es aus Kunststoff besteht und als Kunststoffspritzgussteil ausgebildet ist.

15. Applikationsgefäß nach einem der vorhergehenden Ansprüche, **dadurch gekennzeichnet, dass** der Innendurchmesser des Spritzenzylinders (5) kleiner ist als der Außendurchmesser des Luer-Kegels (4).
